# EUROPEAN PATENT APPLICATION

(11) **EP 3 138 463 A1**
(43) Date of publication of application: **08.03.2017**
(21) Application number: 15786469.5
(22) Date of filing: 21.01.2015
(51) Int. Cl.: A61B 1/00, G02B 23/26

(54) **AID FOR MOUNTING AND DEMOUNTING OPTICAL ADAPTOR FOR ENDOSCOPE**

(30) Priority: 01.05.2014 JP 2014094882
(71) Applicant: OLYMPUS CORPORATION, Hachioji-shi Tokyo 192-8507 (JP)
(72) Inventor: KATO Takahiko, Tokyo 192-8507 (JP)
(74) Representative: Schicker, Silvia
(86) International application number: PCT/JP2015/051543
(87) International publication number: WO 2015/166674

(57) **Abstract**

An endoscope optical adapter attachment/detachment assisting tool 30 includes: an assisting tool body 31 configured to contain and hold an optical adapter 20 attachable to and detachable from a distal end portion 11 of an insertion portion 2 of an endoscope 10; an adapter seizing portion 33a, 36, 43 configured to come into contact with the optical adapter 20 contained in the assisting tool body 31 and press the optical adapter 20 in a direction orthogonal to a direction of attaching and detaching the optical adapter 20 to and from the distal end portion 11 to seize the optical adapter 20; and an urging member 33, 39, 46 configured to urge an adapter holding portion.

## Description

### Technical Field

The present invention relates to an endoscope optical adapter attachment/detachment assisting tool for attaching and detaching an optical adapter to and from a distal end portion of an insertion portion of an endoscope.

### Background Art

Recently, insertion equipment to be inserted into a subject, for example, an endoscope, is widely used in a medical field and an industrial field. In the case of an endoscope used in the medical field, it is possible to, by inserting an elongated insertion portion into a body cavity of a subject, observe an inside of the body cavity and, if necessary, perform various kinds of treatment and the like using a treatment instrument inserted in a treatment instrument insertion channel provided in the endoscope.

Further, in the case of an endoscope used in the industrial field, it is possible to, by inserting an elongated insertion portion of the endoscope into an object such as an inside of a jet engine and piping of a factory, perform observation, various kinds of treatment, inspection and the like for scratches, corrosion and the like of a region to be inspected in the object.

Especially, as the endoscope in the industrial field, an adapter-type endoscope provided with an optical adapter attachable to and detachable from a distal end portion of the insertion portion is known. There are a plurality of kinds of optical adapters used for the adapter-type endoscope, and the optical adapters can change optical characteristics such as a view angle, an observation direction and the like by being attached to the distal end portion of the insertion portion of the endoscope.

A plurality of kinds of such optical adapters exist, and it becomes possible to change the view angle and the observation direction of the endoscope by attaching various kinds of optical adapters. Conventionally, at the time of attaching an optical adapter, the optical adapter is screwedly fixed to the distal end portion of the endoscope by a retaining ring, which is a ring-shaped member provided on the optical adapter, being held by a person's fingers.

However, due to recent downsizing, diameter reduction and the like of optical adapters, it is difficult to perform work of attaching and detaching an optical adapter to and from the distal end portion of the endoscope. Especially, an optical adapter for performing observation in a lateral direction is in a situation that a finger touches a lens of an observation optical system and the like each time the retaining ring of the optical adapter is rotated, and cleaning work is required each time the optical adapter is attached.

As a countermeasure for the situation, a technique for improving workability at the time of attachment by using an assisting tool which can grasp an outer circumference of an optical adapter is known as disclosed in U.S. Patent No. US2005/0050707A1.

However, the conventional assisting tool described in U.S. Patent No. US2005/0050707A1 has a problem that, since an optical adapter is held by an internal O-shaped ring, the O-shaped ring is worn away, and holding force reduces when attachment and detachment of the optical adapter to/from a distal end portion of an endoscope is repeatedly performed.

Thus, in the case of the conventional assisting tool, there is also a possibility that, by reduction in holding force due to deterioration of the O-shaped ring, the optical adapter comes off from inside the assisting tool and is lost.

By the way, in the conventional assisting tool, an amount of tightening force at the time of attaching the optical adapter to the distal end portion of the endoscope depends on frictional force between the O-shaped ring and the retaining ring. Therefore, in the conventional assisting tool, the amount of force of tightening the optical adapter to the distal end portion of the endoscope easily varies depending on a deterioration state of the O-shaped ring, and there is also a possibility that the retaining ring loosens during an inspection, and the optical adapter falls off from the distal end portion and drops into an object.

Therefore, the present invention has been made in view of the above situation, and an object is to propose an endoscope optical adapter attachment/detachment assisting tool capable of easily attaching an optical adapter to a distal end portion of an endoscope and capable of attaching the optical adapter to the distal end portion with a steady amount of force by preventing reduction in holding force internally holding the optical adapter even if attachment/detachment of the optical adapter is repeatedly performed.

### Disclosure of Invention

### Means for Solving the Problem

An endoscope optical adapter attachment/detachment assisting tool of an aspect of the present invention includes: a substantially cylindrical assisting tool body configured to contain and hold an optical adapter attachable to and detachable from a distal end portion of an insertion portion of an endoscope; an adapter seizing portion configured to come into contact with the optical adapter contained in the assisting tool body and press the optical adapter in a direction orthogonal to a direction of attaching and detaching the optical adapter to and from the distal end portion to seize the optical adapter; and an urging member configured to urge the adapter holding portion.

According to the present invention described above, it is possible to provide an endoscope optical adapter attachment/detachment assisting tool capable of easily attaching an optical adapter to a distal end portion of an endoscope and capable of attaching the optical adapter to the distal end portion with a steady amount of force by preventing reduction in holding force internally holding the optical adapter even if attachment/detachment of the optical adapter is repeatedly performed.

### Brief Description of the Drawings

Fig. 1 relates to a first embodiment and is a diagram showing a configuration of an endoscope, an optical adapter and an endoscope optical adapter attachment/detachment assisting tool;
Fig. 2 relates to the first embodiment and is a perspective view showing a configuration of the optical adapter;
Fig. 3 relates to the first embodiment and is a cross-sectional view showing the configuration of the optical adapter;
Fig. 4 relates to the first embodiment and is a partial cross-sectional view showing a state that the optical adapter is attached to a distal end portion of the endoscope;
Fig. 5 relates to the first embodiment and is a perspective view showing a configuration of the endoscope optical adapter attachment/detachment assisting tool;
Fig. 6 relates to the first embodiment and is a cross-sectional view showing the configuration of the endoscope optical adapter attachment/detachment assisting tool;
Fig. 7 relates to the first embodiment and is a cross-sectional view showing the configuration of the endoscope optical adapter attachment/detachment assisting tool in a state that the optical adapter is contained and held;
Fig. 8 relates to the first embodiment and is an enlarged view of a circle VIII portion in Fig. 7;
Fig. 9 relates to the first embodiment and is a partial cross-sectional view showing a state before the optical adapter is attached to the distal end portion of an insertion portion of the endoscope by the endoscope optical adapter attachment/detachment assisting tool;
Fig. 10 relates to the first embodiment and is a partial cross-sectional view showing a state that the optical adapter is attached to the distal end portion of the insertion portion of the endoscope by the endoscope optical adapter attachment/detachment assisting tool;
Fig. 11 relates to the first embodiment and is a partial cross-sectional view showing a state that the endoscope optical adapter attachment/detachment assisting tool has been detached from the distal end portion of the insertion portion of the endoscope to which the optical adapter is attached;
Fig. 12 relates to the first embodiment, shows a first modification, and is a plan view showing an example of a tapered face formed on a recess portion of a retaining ring of the optical adapter;
Fig. 13 relates to the first embodiment, shows a second modification, and is a partial cross-sectional view showing a configuration in which a tapered face is formed on a lock pin of a plunger of the endoscope optical adapter attachment/detachment assisting tool;
Fig. 14 relates to a second embodiment and is a partial cross-sectional view showing a state before an optical adapter is attached to a distal end portion of an insertion portion of an endoscope by an endoscope optical adapter attachment/detachment assisting tool;
Fig. 15 relates to the second embodiment and is a cross-sectional view along a XV-XV line in Fig. 14;
Fig. 16 relates to the second embodiments and is a partial cross-sectional view showing a state that the optical adapter is attached to the distal end portion of the insertion portion of the endoscope by the endoscope optical adapter attachment/detachment assisting tool;
Fig. 17 relates to the second embodiments and is a partial cross-sectional view showing a state that the endoscope optical adapter attachment/detachment assisting tool has been detached from the distal end portion of the insertion portion of the endoscope to which the optical adapter is attached;
Fig. 18 relates to a third embodiment and is a partial cross-sectional view showing a state before an optical adapter is attached to a distal end portion of an insertion portion of an endoscope by an endoscope optical adapter attachment/detachment assisting tool;
Fig. 19 relates to the third embodiment and is a cross-sectional view along a XIX-XIX line in Fig. 18;
Fig. 20 relates to the third embodiment and is a partial cross-sectional view showing a state that the optical adapter is attached to the distal end portion of the insertion portion of the endoscope by the endoscope optical adapter attachment/detachment assisting tool;
Fig. 21 relates to the third embodiment and is a partial cross-sectional view showing a state that the endoscope optical adapter attachment/detachment assisting tool has been detached from the distal end portion of the insertion portion of the endoscope to which the optical adapter is attached;
Fig. 22 is a perspective view showing a configuration of an endoscope optical adapter attachment/detachment assisting tool of a first reference example;
Fig. 23 is a cross-sectional view showing the configuration of the endoscope optical adapter attachment/detachment assisting tool of the first reference example;
Fig. 24 is a perspective view showing a configuration of an endoscope optical adapter attachment/detachment assisting tool of a second reference example; and
Fig. 25 is a cross-sectional view showing the configuration of the endoscope optical adapter attachment/detachment assisting tool of the second reference example.

### Best Mode for Carrying Out the Invention

Preferable embodiments of the present invention will be described below with reference to drawings. Note that, in each figure used in the description below, a different reduced scale is used for each component so that the component is shown in a size recognizable on a drawing, and the present invention is not limited only to the number of components, shapes of the components, a ratio of sizes of the components and relative positional relationships among the respective components shown in the figures. Further, in the description below, there may be a case where description is made on an assumption that upward and downward directions at time of facing a drawing correspond to upper and lower parts of a component, respectively.

### (First Embodiment)

First, an optical unit and an endoscope of a first embodiment of the present invention will be described below based on drawings.

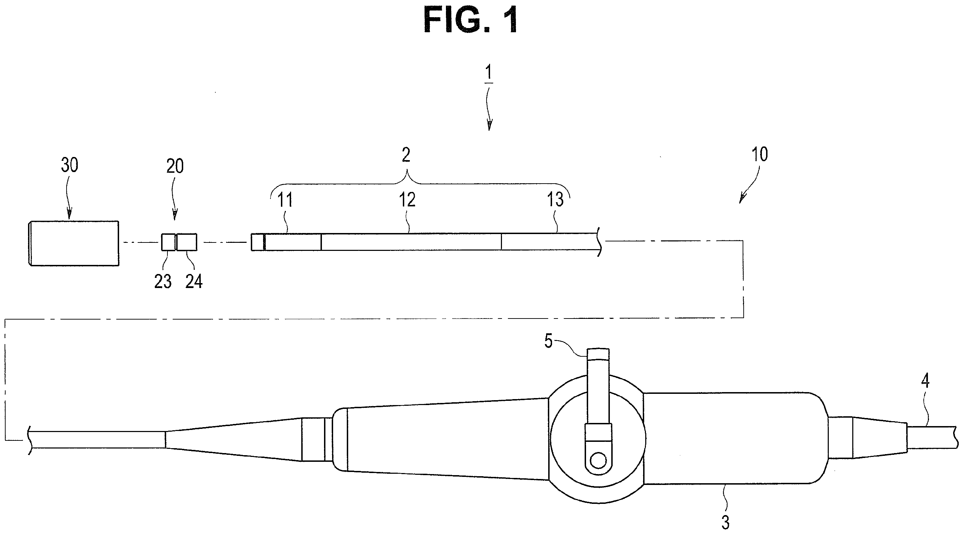

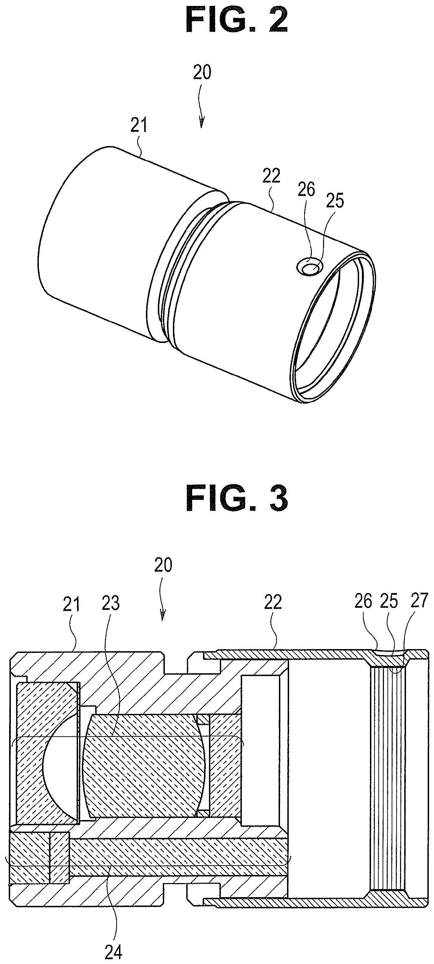

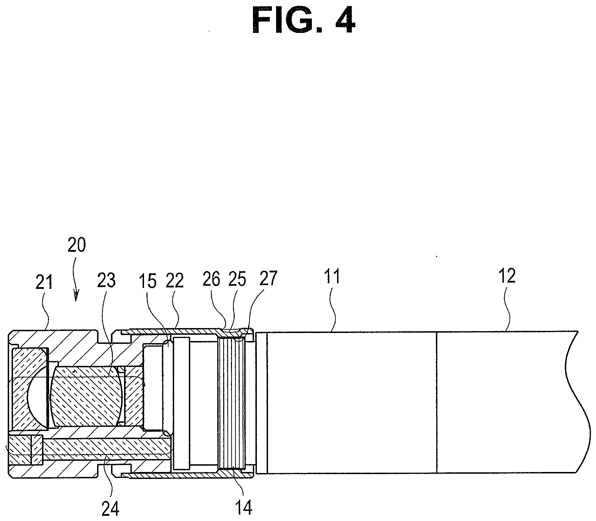

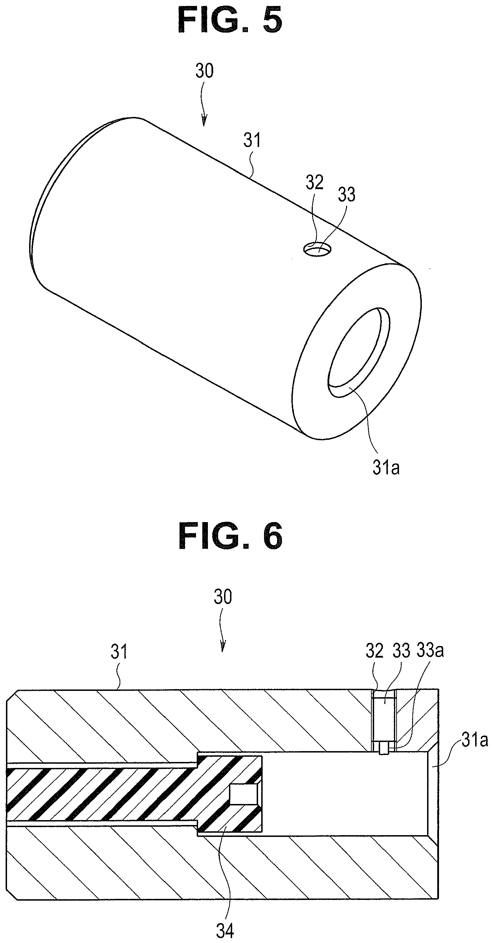

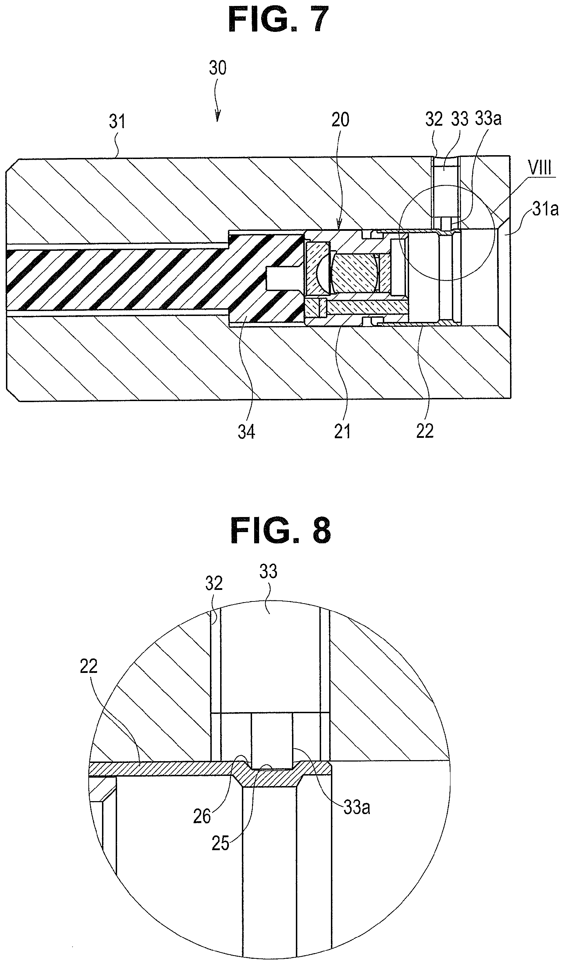

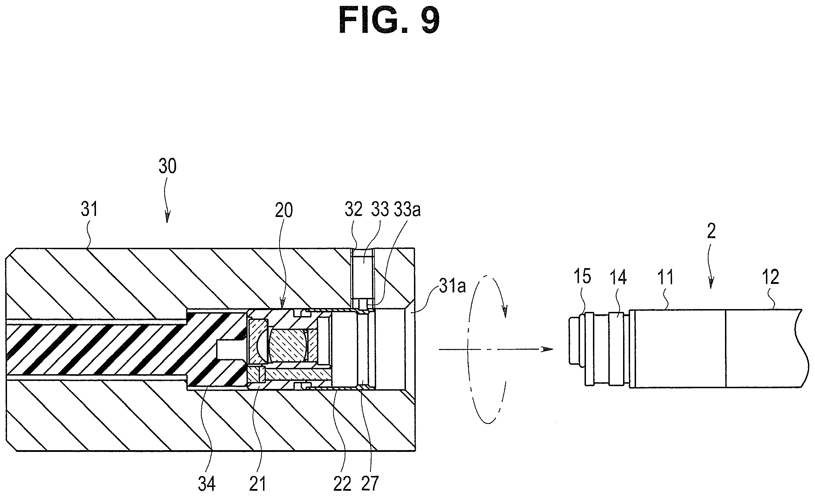

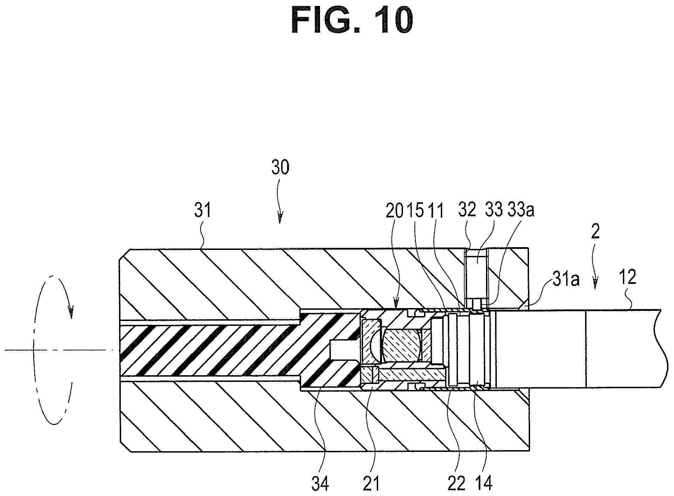

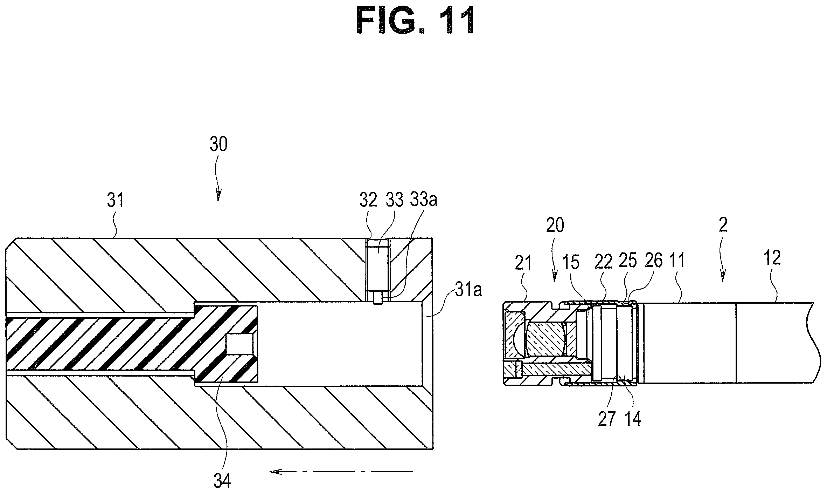

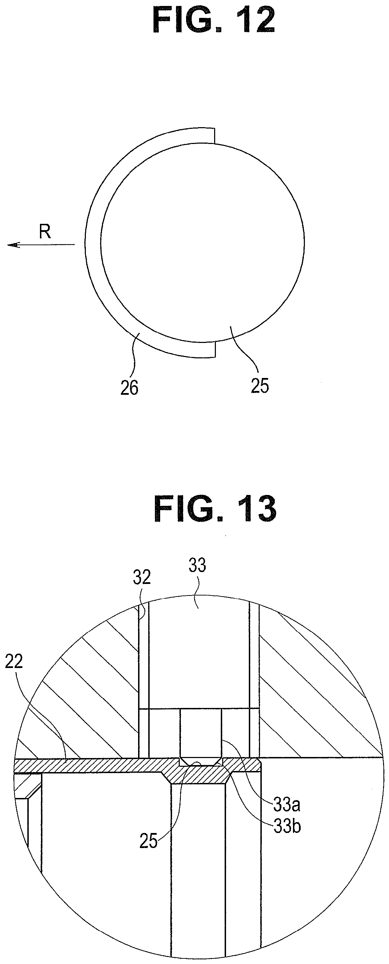

Note that Fig. 1 is a diagram showing a configuration of an endoscope, an optical adapter and an endoscope optical adapter attachment/detachment assisting tool; Fig. 2 is a perspective view showing a configuration of the optical adapter; Fig. 3 is a cross-sectional view showing the configuration of the optical adapter; Fig. 4 is a partial cross-sectional view showing a state that the optical adapter is attached to a distal end portion of the endoscope; Fig. 5 is a perspective view showing a configuration of the endoscope optical adapter attachment/detachment assisting tool; Fig. 6 is a cross-sectional view showing the configuration of the endoscope optical adapter attachment/detachment assisting tool; Fig. 7 is a cross-sectional view showing the configuration of the endoscope optical adapter attachment/detachment assisting tool in a state that the optical adapter is contained and held; Fig. 8 is an enlarged view of a circle VIII portion in Fig. 7; Fig. 9 is a partial cross-sectional view showing a state before the optical adapter is attached to the distal end portion of the insertion portion of the endoscope by the endoscope optical adapter attachment/detachment assisting tool; Fig. 10 is a partial cross-sectional view showing a state that the optical adapter is attached to the distal end portion of the insertion portion of the endoscope by the endoscope optical adapter attachment/detachment assisting tool; Fig. 11 is a partial cross-sectional view showing a state that the endoscope optical adapter attachment/detachment assisting tool has been detached from the distal end portion of the insertion portion of the endoscope to which the optical adapter is attached; Fig. 12 shows a first modification, and is a plan view showing an example of a tapered face formed on a recess portion of a retaining ring of the optical adapter; and Fig. 13 shows a second modification, and is a partial cross-sectional view showing a configuration in which a tapered face is formed on a lock pin of a plunger of the endoscope optical adapter attachment/detachment assisting tool.

First, a schematic configuration of the present embodiment will be described.

An endoscope system 1 of the present embodiment is configured having an endoscope 10, an optical adapter 20 and an endoscope optical adapter attachment/detachment assisting tool (hereinafter simply referred to as an assisting tool) 30 as shown in Fig. 1.

First, a configuration of the endoscope 10 of the endoscope system 1 will be described below.

The endoscope 10 is configured being provided with mainly an insertion portion 2 configured to be inserted into a subject, an operation portion 3 provided on a proximal end side of the insertion portion 2, and a cable 4 for connecting the endoscope 10 to an external apparatus not shown which is provided with, for example, a display apparatus, the cable 4 extending from the operation portion 3.

Note that, as the endoscope 10 here, an industrial endoscope, the insertion portion 2 of which is inserted into an object which is a structure such as a machine or a building, is shown as an example, components applicable to a medical endoscope for observing an inside of a living body such as a human body are included.

The insertion portion 2 is configured with a distal end portion 11 arranged at a distal end, a bendable bending portion 12 arranged on a proximal end side of the distal end portion 11, and a deformable flexible tube portion 13 arranged on a proximal end side of the bending portion 12 and connected to a distal end side of the operation portion 3, which are connected to one another. Note that the endoscope 10 may be in a form referred to as a so-called rigid endoscope which does not have the deformable flexible tube portion 13 on the insertion portion 2.

The distal end portion 11 is provided with an image pickup portion and an illuminating light emitting portion not shown. Further, the operation portion 3 is provided with an angle operating lever 5 for operating bending of the bending portion 12.

The cable 4 has a connector portion configured to be attachable to and detachable from the external apparatus though it is not shown. The external apparatus is provided with a power supplying portion configured to supply power to the endoscope 10, an image processing portion configured to process an image picked up by the image pickup portion of the endoscope 10 so that the image can be displayed on the display apparatus, and the like.

Note that, if the endoscope 10 is provided with a battery and has a configuration for wirelessly performing communication with the external apparatus, the cable 4 is unnecessary.

Next, a configuration of the optical adapter 20 of the endoscope system 1 will be described below.

As shown in Fig. 2, the optical adapter 20 is configured having a substantially cylindrical lens holding frame 21 provided on a distal end side, and an annular retaining ring 22 which is rotatably fitted on a proximal end portion of the lens holding frame 21.

As shown in Fig. 3, an objective optical system 23 constituted by a plurality of objective lens groups, and an illumination optical system 24 including an illumination lens and a light guide configured to transmit illuminating light are included in the lens holding frame 21.

Note that a recess portion 25 having a circular section is formed on an outer circumferential portion of the retaining ring 22, and a tapered face 26 is formed on a wall portion forming the recess portion 25. Further, a female screw portion 27 is formed on an inner circumferential portion of the retaining ring 22.

As shown in Fig. 4, the optical adapter 20 is detachably attached by the retaining ring 22 being screwed to the distal end portion 11 of the insertion portion 2 of the endoscope 10 and can change optical characteristics of the endoscope 10.

More specifically, the optical adapter 20 is attached to the distal end portion 11 by the distal end portion 11 of the endoscope 10 being insertedly fitted from an opening of the retaining ring 22, and the female screw portion 27 of the retaining ring 22 being screwed to a male screw portion 14 formed on an outer circumferential portion of the distal end portion 11.

At this time, an O-shaped ring 15 provided on the distal end portion 11 comes into close contact with a proximal end face of the lens holding frame 21 of the optical adapter 20, and the optical adapter 20 is attached to the distal end portion 11 in a state of being watertightly held.

Note that a plurality of kinds of optical adapters 20 exist, and it is possible to change various optical characteristics, such as a view angle, such as close-up photography, wide-angle and enlargement (view from a distance), and an observation direction, such as front view, lateral view and oblique view, according to the kind of the optical adapter 20 to be attached to the distal end portion 11 of the endoscope 10.

Next, a configuration of the assisting tool 30 of the endoscope system 1 will be described below.

As shown in Figs. 5 and 6, the assisting tool 30 is cast in metal such as stainless steel, rigid resin or the like and has a cylindrical assisting tool body 31 configured to contain and hold the optical adapter 20.

On an outer circumferential portion of the assisting tool body 31, a hole portion 32 is formed in an inner diameter center direction, and a plunger (spring plunger) 33 as an urging member is arranged in the hole portion 32.

The plunger 33 is screwed in the hole portion 32 and is arranged so that, by an amount of screw into the hole portion 32 being adjusted, a lock pin 33a as an adapter seizing portion which is urged by an internal coil spring not shown and which can freely project and retreat, projects from an inner circumferential face of the assisting tool body 31 by a predetermined length.

Inside the assisting tool body 31, an axial direction position restricting member 34 for positioning the optical adapter 20 in an axial direction is screwed and provided. Thereby, a distal end of the assisting tool 30 is blocked and forms a cylindrical shape having a bottom and having, at a proximal end, an opening 31a where a tapered face is formed.

The optical adapter 20 is inserted into the assisting tool 30 from the opening 31a side of the assisting tool body 31, and the assisting tool 30 contains and holds the optical adapter 20 in a state that the optical adapter 20 is inserted up to a position where a distal end face is in contact with a proximal end face of the axial direction position restricting member 34 as shown in Fig. 7.

Note that, as shown in Fig. 8, a position of the optical adapter 20 contained in the assisting tool 30 around a longitudinal axis is adjusted so that the lock pin 33a of the plunger 33 is fitted in the recess portion 25 formed on the retaining ring 22 and seizes the retaining ring 22.

Thus, the optical adapter 20 is held in the assisting tool body 31 of the assisting tool 30 in a state of being pressed in an inner diameter direction orthogonal to a longitudinal direction, by the lock pin 33a of the plunger 33 being fitted in the recess portion 25 of the retaining ring 22. Thereby, the optical adapter 20 is prevented from falling off from the assisting tool body 31 of the assisting tool 30.

Next, description will be made on a procedure for attaching and detaching the optical adapter 20 contained and held in the assisting tool 30 to and from the distal end portion 11 of the insertion portion 2 to change the optical characteristics of the endoscope 10, such as a view angle, such as close-up photography, wide-angle and enlargement (view from a distance), an observation direction and the like by the assisting tool 30 of the present embodiment configured as described above at time of using the endoscope 10.

First, in a state that the optical adapter 20 is contained and held in the assisting tool body 31 as shown in Fig. 9, the assisting tool 30 is attached by a user so as to enclose the distal end portion 11 of the insertion portion 2 of the endoscope 10 from the opening 31a on the assisting tool body 31 side.

Then, the assisting tool 30 is rotated in a predetermined direction, and the optical adapter 20 is attached to the distal end portion 11 by the female screw portion 27 of the retaining ring 22 of the optical adapter 20 in the assisting tool body 31 and the male screw portion 14 of the distal end portion 11 being screwed to each other as shown in Fig. 10.

At this time, the lock pin 33a of the plunger 33 provided on the assisting tool body 31 of the assisting tool 30 is in a state of projecting in a direction orthogonal to a direction of attaching and detaching the optical adapter 20 to or from the distal end portion 11 and being fitted in the recess portion 25 of the retaining ring 22 and seizing the retaining ring 22.

That is, the lock pin 33a of the plunger 33 presses and seizes the retaining ring 22 in the direction orthogonal to the direction of attaching and detaching the optical adapter 20 to and from the distal end portion 11.

Therefore, by being seized by the plunger 33, the optical adapter 20 is contained in the assisting tool body 31 of the assisting tool 30 with sufficient holding force without running on idle, against rotation torque at the time of screwing the retaining ring 22 of the optical adapter 20 and the distal end portion 11 of the endoscope 10 to each other.

Thereby, the user can certainly fasten the retaining ring 22 of the optical adapter 20 to the distal end portion 11 by rotating the assisting tool 30 in the predetermined direction.

Then, after the retaining ring 22 of the optical adapter 20 is screwed to the distal end portion 11 and cannot rotate any more, the lock pin 33a of the plunger 33 retreats into the plunger 33 along a tapered face 26 formed on the recess portion 25 and comes off from the recess portion 25, and the optical adapter 20 enters an unseized state in the assisting tool body 31 of the assisting tool 30.

By the assisting tool 30 being pulled in a direction away from the distal end portion 11 in this state, the optical adapter 20 attached to the distal end portion 11 is caused to leave the assisting tool body 31 as shown in Fig. 11. Thus, the optical adapter 20 can be easily attached to the distal end portion 11 of the endoscope 10.

In this way, by the optical adapter 20 being attached to the distal end portion 11 of the insertion portion 2, the optical characteristics of the endoscope 10, such as a view angle, such as close-up photography, wide-angle and enlargement (view from a distance), and an observation direction are changed according to the kind of the attached optical adapter 20.

Note that, at the time of removing the optical adapter 20 from the distal end portion 11 of the insertion portion 2 of the endoscope 10, the optical adapter 20 attached to the distal end portion 11 is removed with use of the assisting tool 30 in a procedure opposite to the procedure at the time of attaching the optical adapter 20, and the optical adapter 20 is contained and held in the assisting tool body 31 of the assisting tool 30.

More specifically, the user encloses the optical adapter 20 attached to the distal end portion 11 of the insertion portion 2 of the endoscope 10 into the assisting tool body 31 from the opening 31a side of the assisting tool body 31 of the assisting tool 30.

At this time, the assisting tool 30 encloses the optical adapter 20 up to a position where the distal end face of the optical adapter 20 comes into contact with the proximal end face of the axial direction position restricting member 34, and is rotation-adjusted to such a position that the lock pin 33a of the plunger 33 is fitted in the recess portion 25 of the retaining ring 22 and seizes the retaining ring 22.

In this way, the assisting tool 30 enters a state that the lock pin 33a of the plunger 33 seizes the retaining ring 22 of the optical adapter 20 with holding force enough to prevent the optical adapter 20 from running on idle, against rotation torque required at time of loosening the screwed state between the retaining ring 22 of the optical adapter 20 and the distal end portion 11.

In this state, the assisting tool 30 is rotated in a direction opposite to the predetermined direction at the time of attachment to the distal end portion 11 of the endoscope 10 described above, and the screwed state between the female screw portion 27 of the retaining ring 22 of the optical adapter 20 in the assisting tool body 31 and the male screw portion 14 of the distal end portion 11 is loosened, so that the screwed state between the retaining ring 22 and the distal end portion 11 is released.

Then, by the assisting tool 30 being pulled in the direction away from the distal end portion 11, the optical adapter 20 is removed from the distal end portion 11, and the optical adapter 20 is contained and held in the assisting tool body 31 in the state that the lock pin 33a of the plunger 33 seizes the retaining ring 22.

As described above, the assisting tool 30 is configured to contain and hold the optical adapter 20 in the assisting tool body 31 by inserting the optical adapter 20 up to the axial direction position restricting member 34 of the assisting tool body 31, and the lock pin 33a of the plunger 33 seizing the retaining ring 22, being fitted in the recess portion 25 of the retaining ring 22.

Therefore, the optical adapter 20 contained and held in the assisting tool body 31 is prevented from running on idle by being seized by the plunger 33 even if the rotation torque required at the time of screwingly attaching or detaching the retaining ring 22 to or from the distal end portion 11 is applied at the time of being attached to or detaching from the distal end portion 11 of the insertion portion 2 of the endoscope 10, and the optical adapter 20 can be easily attached to or detached from the distal end portion 11 with a predetermined amount of force.

Furthermore, the assisting tool 30 has an advantage that it is possible to prevent loss of the optical adapter 20 because the assisting tool 30 contains and holds the optical adapter 20 when the optical adapter 20 is not used without being attached to the distal end portion 11 of the endoscope 10.

From the above description, since the assisting tool 30 of the present embodiment is in a configuration that the optical adapter 20 inside the assisting tool 30 is seized by the plunger 33, abrasion, deterioration and the like of the plunger 33 do not easily occur even if the optical adapter 20 is repeatedly attached to and detached from the distal end portion 11 of the endoscope 10, and, therefore, the assisting tool 30 is in a configuration that the force of holding the optical adapter 20 is prevented from decreasing.

Therefore, the assisting tool 30 is in a configuration that the optical adapter 20 does not easily come off from the assisting tool body 31 because the lock pin 33a of the plunger 33 is fitted in the recess portion 25 of the retaining ring 22 to maintain a seized state, and the assisting tool 30 can prevent loss of the optical adapter 20.

Furthermore, by the force of holding the optical adapter 20 being prevented from decreasing, an amount of fastening or loosening force at the time of attaching or detaching the optical adapter 20 to or from the distal end portion 11 of the endoscope 10 becomes constant, and, therefore, the assisting tool 30 can stably attach or detach the optical adapter 20 to or from the distal end portion 11.

Especially, since fastening the optical adapter 20 to the distal end portion 11 of the endoscope 10 can be stably performed, it does not happen that the retaining ring 22 loosens during an inspection, and the optical adapter 20 is prevented from falling off from the distal end portion 11 into an object.

From the above, the assisting tool 30 of the present embodiment is configured to be capable of easily attaching the optical adapter 20 to the distal end portion 11 of the insertion portion 2 of the endoscope 10, and capable of attaching the optical adapter 20 to the distal end portion 11 with a stable amount of force, preventing the force of internally holding the optical adapter 20 from decreasing even if attachment and detachment of the optical adapter 20 is repeatedly performed.

### (First Modification)

Note that the optical adapter 20 can appropriately adjust the holding force by the lock pin 33a of the plunger 33 of the assisting tool 30 by changing an angle, a direction and the like of the tapered face 26 provided around the recess portion 25 formed on the retaining ring 22, and can set the amount of holding force at the time of attaching and detaching the optical adapter 20 to and from the distal end portion 11 by the assisting tool 30 can be set.

As an example of the above, a configuration is also possible in which the tapered face 26 is provided only on a direction R side of the recess portion 25 where the retaining ring 22 is rotated at the time of attaching the optical adapter 20 to the distal end portion 11 of the insertion portion 2 of the endoscope 10, for example, as shown in Fig. 12.

According to such a configuration, since the lock pin 33a of the plunger 33 of the assisting tool 30 comes into contact with a wall face of the recess portion 25 where the tapered face 26 is not provided, at the time of attaching the optical adapter 20 to the distal end portion 11, the holding force by the lock pin 33a can be increased. Therefore, it becomes difficult for the lock pin 33a to come off from the recess portion 25, and it is possible to increase the amount of the holding force at the time of attaching the optical adapter 20 to the distal end portion 11 by the assisting tool 30.

### (Second Modification)

Further, as shown in Fig. 13, a configuration is also possible in which the tapered face 26 is not provided on the recess portion 25, and a tapered face 33b is provided on the lock pin 33a side of the plunger 33 of the assisting tool 30.

### (Second Embodiment)

Next, an optical unit and an endoscope of a second embodiment of the present invention will be described below based on drawings.

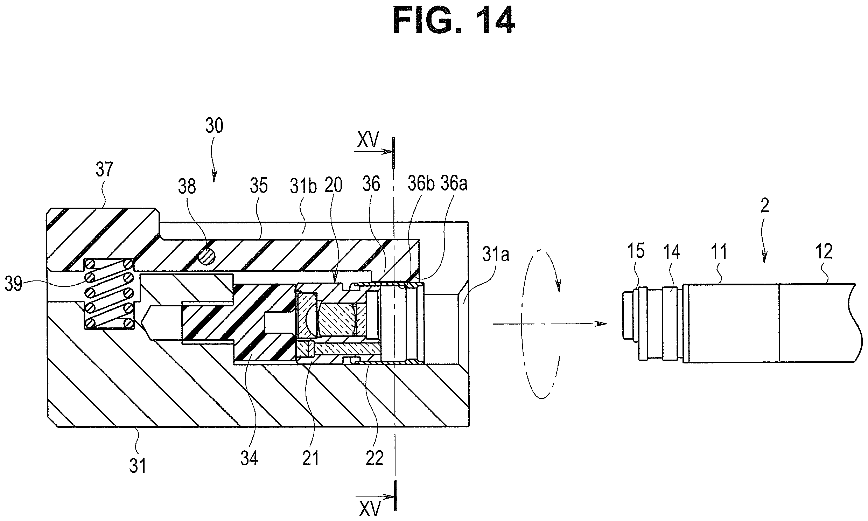

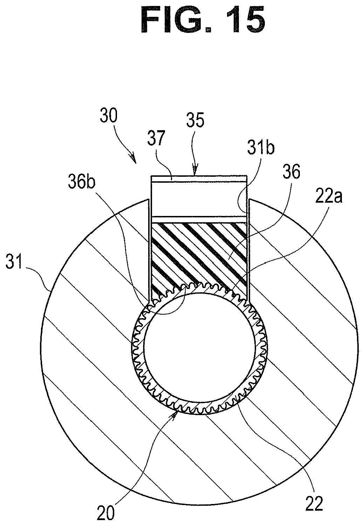

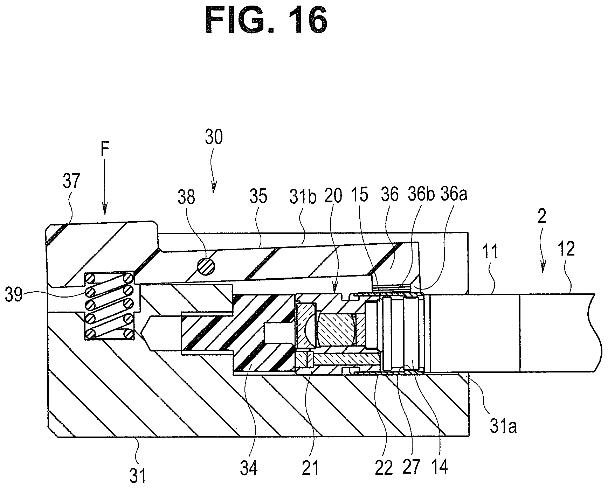

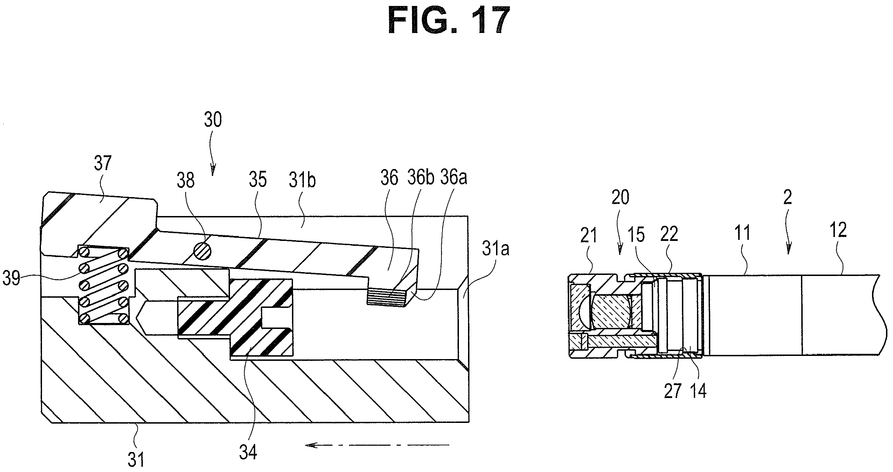

Fig. 14 is a partial cross-sectional view showing a state before an optical adapter is attached to a distal end portion of an endoscope by an endoscope optical adapter attachment/detachment assisting tool; Fig. 15 is a cross-sectional view along a XV-XV line in Fig. 14; Fig. 16 is a partial cross-sectional view showing a state that the optical adapter is attached to the distal end portion of the insertion portion of the endoscope by the endoscope optical adapter attachment/detachment assisting tool; and Fig. 17 is a partial cross-sectional view showing a state that the endoscope optical adapter attachment/detachment assisting tool has been detached from the distal end portion of the insertion portion of the endoscope to which the optical adapter is attached.

Note that, in the description below, same reference numerals will be used for same components as described in the first embodiment described above, and detailed description of the components will be omitted.

In the assisting tool 30 of the present embodiment, a structure for holding and fixing the optical adapter 20 in the assisting tool body 31 is in a lever-type configuration unlike the plunger 33 of the first embodiment.

More specifically, the assisting tool 30 is provided with a holding lever 35 as an adapter seizing member configured to hold the optical adapter 20 inside the assisting tool body 31 as shown in Figs. 14 and 15.

The holding lever 35 is formed with metal or rigid resin and provided in a groove portion 31b along a longitudinal direction which is formed on the assisting tool body 31 of the assisting tool 30.

The holding lever 35 is rotatably held in the groove portion 31b by a pin member 38 and has an adapter seizing portion 36 configured to come into contact with the retaining ring 22 of the optical adapter 20, on a proximal end portion, and an operation projection portion 37 projecting so that the user can press it, on a distal end portion.

Further, the assisting tool body 31 is provided with a coil spring 39 as an urging member configured to urge the distal end portion of the holding lever 35 to be on the operation projection portion 37 side, in an outer diameter direction. Therefore, the holding lever 35 is caused to be in a state of projecting from the groove portion 31b of the assisting tool body 31 by urging force of the coil spring 39, and is caused to rotate around the pin member 38 by the operation projection portion 37 being pressed into the assisting tool body 31 by the user.

Note that, on a proximal end of the adapter seizing portion 36 of the holding lever 35, a tapered face 36a is formed so that the optical adapter 20 is easily introduced into the assisting tool body 31. As shown in Fig. 15, the adapter seizing portion 36 has a holding face 36b with a recessedly curved section, on which recesses and projections to be engaged with a knurling 22a formed on an outer circumference of the retaining ring 22 of the optical adapter 20 are formed.

In the assisting tool 30 configured as described above, in the state that the optical adapter 20 is contained in the assisting tool body 31, the holding lever 35 holds the optical adapter 20 by the urging force of the coil spring 39, by the holding face 36b of the adapter seizing portion 36 being in contact with the retaining ring 22 of the optical adapter 20, pressing the retaining ring 22.

The assisting tool 30 is configured to hold the optical adapter 20 in such a manner that the retaining ring 22 is prevented from running on idle against the rotation torque required at the time of screwing the retaining ring 22 of the optical adapter 20 to the distal end portion 11 of the endoscope 10, by the holding face 36b, on which the recesses and projections are formed, being engaged with the knurling 22a of the retaining ring 22 of the optical adapter 20. Note that the optical adapter 20 here is in a configuration that the recess portion 25 is not formed on the retaining ring 22.

Next, description will be made below on a procedure for attaching and detaching the optical adapter 20 contained and held in the assisting tool 30 to and from the distal end portion 11 of the insertion portion 2 to change the optical characteristics of the endoscope 10, such as a view angle, such as close-up photography, wide-angle and enlargement (view from a distance), and an observation direction, by the assisting tool 30 of the present embodiment configured as described above at the time of using the endoscope 10.

First, in a state that the optical adapter 20 is contained and held in the assisting tool body 31 shown in Figs. 14 and 15, the assisting tool 30 is attached by the user so as to enclose the distal end portion 11 of the insertion portion 2 of the endoscope 10 from the opening 31a on the assisting tool body 31 side.

Then, the assisting tool 30 is rotated in the predetermined direction, and the optical adapter 20 is attached to the distal end portion 11 by the female screw portion 27 of the retaining ring 22 of the optical adapter 20 in the assisting tool body 31 and the male screw portion 14 of the distal end portion 11 being screwed to each other as shown in Fig. 16.

Note that, at the time of attaching the optical adapter 20 to the distal end portion 11, the holding face 36b of the adapter seizing portion 36 provided on the holding lever 35 of the assisting tool 30 is in the state of being engaged with the knurling 22a of the retaining ring 22.

At this time, the adapter seizing portion 36 provided on the assisting tool body 31 of the assisting tool 30 is in a state of seizing the optical adapter 20, pressing the retaining ring 22 in the direction orthogonal to the direction of attaching and detaching the optical adapter 20 to and from the distal end portion 11. That is, the adapter seizing portion 36 presses and seizes the retaining ring 22 in the direction orthogonal to the direction of attaching and detaching the optical adapter 20 to and from the distal end portion 11.

Therefore, the optical adapter 20 is contained in the assisting tool body 31 of the assisting tool 30 with sufficient holding force without the retaining ring 22 engaged with the holding face 36b running on idle, against rotation torque at the time of screwing the retaining ring 22 of the optical adapter 20 and the distal end portion 11 of the endoscope 10 to each other.

Thereby, the user can certainly fasten the retaining ring 22 of the optical adapter 20 to the distal end portion 11 by rotating the assisting tool 30 in the predetermined direction.

In the assisting tool 30, when the operation projection portion 37 of the holding lever 35 is pressed in an inner diameter direction (a direction indicated by an arrow F in Fig. 16) against the urging force of the coil spring 39 in this state, the holding lever 35 rotates around the pin member 38, and the adapter seizing portion 36 leaves the retaining ring 22 of the optical adapter 20.

Then, by the assisting tool 30 being pulled in the direction away from the distal end portion 11, the optical adapter 20 attached to the distal end portion 11 is caused to leave the assisting tool body 31 as shown in Fig. 17. Thus, the optical adapter 20 can be easily attached to the distal end portion 11 of the endoscope 10.

In this way, by the optical adapter 20 being attached to the distal end portion 11 of the insertion portion 2, the optical characteristics of the endoscope 10, such as a view angle, such as close-up photography, wide-angle and enlargement (view from a distance), and an observation direction are changed according to the kind of the attached optical adapter 20.

Note that, at the time of removing the optical adapter 20 from the distal end portion 11 of the insertion portion 2 of the endoscope 10, the optical adapter 20 attached to the distal end portion 11 is removed with use of the assisting tool 30 in a procedure opposite to the procedure at the time of attaching the optical adapter 20, and the optical adapter 20 is contained and held in the assisting tool body 31 of the assisting tool 30.

The assisting tool 30 of the present embodiment configured as described above has effects similar to those of the first embodiment and has an advantage that it is possible to prevent loss of the optical adapter 20 because the assisting tool 30 contains and holds the optical adapter 20 when the optical adapter 20 is not used without being attached to the distal end portion 11 of the endoscope 10.

Further, since the assisting tool 30 is configured so that the adapter seizing portion 36 provided on the holding lever 35 receives the urging force of the coil spring 39 and holds the internal optical adapter 20, pressing the optical adapter 20, the coil spring 39 and the adapter seizing portion 36 provided on the holding lever 35 do not easily wear out, deteriorate or the like even if the optical adapter 20 is repeatedly attached to and detached from the distal end portion 11 of the endoscope 10. Therefore, the assisting tool 30 is configured so that decrease in the force of holding the optical adapter 20 is prevented.

Therefore, the assisting tool 30 is configured such that the optical adapter 20 does not easily fall off from the assisting tool body 31 because the state of the adapter seizing portion 36 provided on the holding lever 35 being engaged with the knurling 22a of the retaining ring 22, and pressing the knurling 22a is kept. Thereby, it is possible to prevent loss of the optical adapter 20.

Furthermore, here also, by the force of holding the optical adapter 20 being prevented from decreasing, the amount of fastening or loosening force at the time of attaching or detaching the optical adapter 20 to or from the distal end portion 11 of the endoscope 10 becomes constant, and, therefore, the assisting tool 30 can stably attach or detach the optical adapter 20 to or from the distal end portion 11. Thereby, it does not happen that the retaining ring 22 of the optical adapter 20 loosens during an inspection, and the optical adapter 20 is prevented from falling off from the distal end portion 11 and dropping into an object.

From the above, similar to the first embodiment, the assisting tool 30 of the present embodiment is also configured to be capable of easily attaching the optical adapter 20 to the distal end portion 11 of the insertion portion 2 of the endoscope 10, and capable of attaching the optical adapter 20 to the distal end portion 11 with a stable amount of force, preventing the force of internally holding the optical adapter 20 from decreasing even if attachment and detachment of the optical adapter 20 is repeatedly performed.

### (Third Embodiment)

Next, an optical unit and an endoscope of a third embodiment of the present invention will be described below based on drawings.

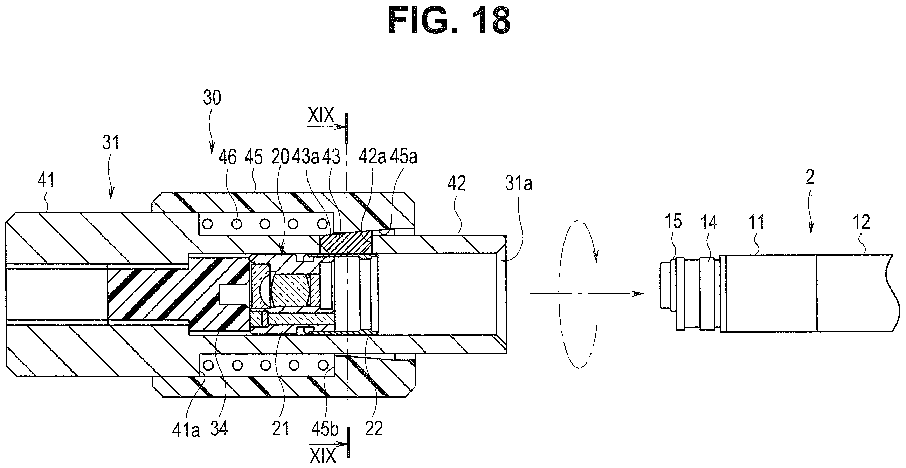

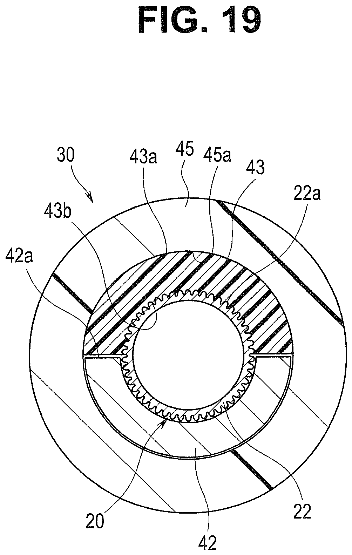

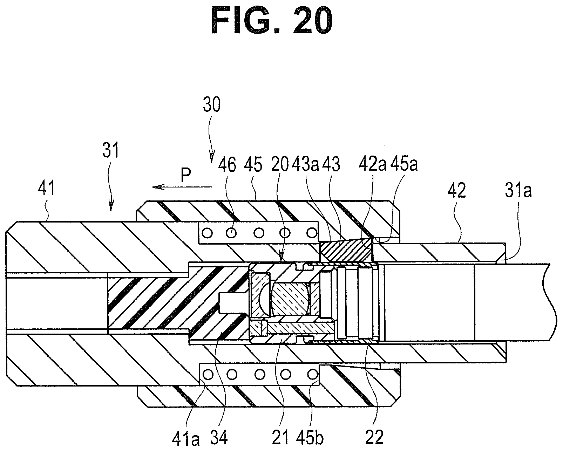

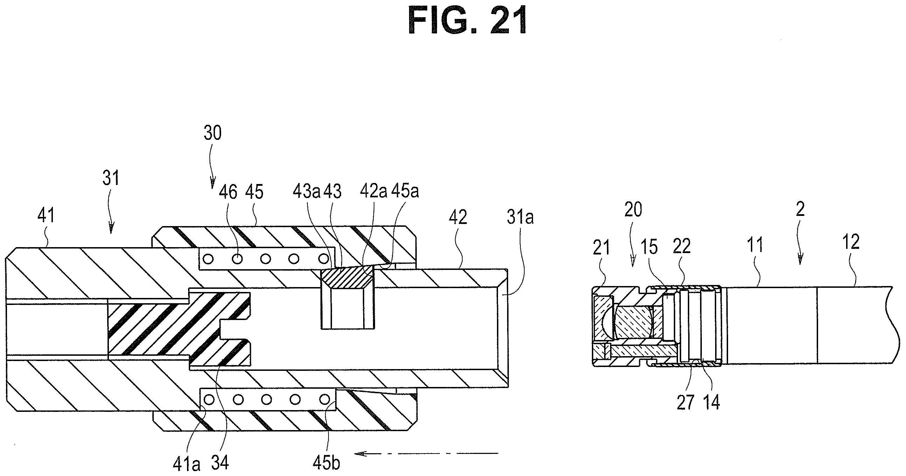

Fig. 18 is a partial cross-sectional view showing a state before an optical adapter is attached to a distal end portion of an endoscope by an endoscope optical adapter attachment/detachment assisting tool; Fig. 19 is a cross-sectional view along a XIX-XIX line in Fig. 18; Fig. 20 is a partial cross-sectional view showing a state that the optical adapter is attached to the distal end portion of an insertion portion of the endoscope by the endoscope optical adapter attachment/detachment assisting tool; and Fig. 21 is a partial cross-sectional view showing a state that the endoscope optical adapter attachment/detachment assisting tool has been detached from the distal end portion of the insertion portion of the endoscope to which the optical adapter is attached.

Note that, in the description below, same reference numerals will be used for same components as described in the first and second embodiments described above, and detailed description of the components will be omitted.

In the assisting tool 30 of the present embodiment also, the structure for holding and fixing the optical adapter 20 in the assisting tool body 31 is different from the plunger 33 of the first embodiment and the holding lever 35 of the second embodiment.

In the assisting tool 30 here, the assisting tool body 31 has a larger diameter portion 41 positioned on a distal end side and a small diameter portion 42 integrally formed with the larger diameter portion 41 and positioned on a proximal end side as shown in Fig. 18.

On a midway portion of the small diameter portion 42 of the assisting tool body 31, a notch portion 42a with a semicircular section is formed up to a middle position from an outer circumferential direction (see Fig. 19), and an adapter seizing portion 43 with a semicircular section and in a shape similar to the shape of the notch portion 42a is arranged.

The adapter seizing portion 43 is formed with metal or rigid resin and has such a tapered face 43 a that a diameter of an outer circumferential face increases in a proximal end direction and a holding face 43b with a recessedly curved section, on which recesses and projections to be engaged with the knurling 22a formed on the outer circumference of the retaining ring 22 of the optical adapter 20 are formed, on an inner circumferential face (see Fig. 19).

On the outer circumferential portion of the assisting tool body 31, a cylindrical lock tube 45 formed with metal or rigid resin is arranged movably back and forth, enclosing the outer circumferential portion. The lock tube 45 is continuously urged in a proximal end direction by a coil spring 46 as an urging member enclosing the small diameter portion 42.

Note that the coil spring 46 continuously urges the lock tube 45 in the proximal end direction by an distal end being in contact with a contact face 41a to be a proximal end face of the larger diameter portion 41 of the assisting tool body 31 and a proximal end being in contact with a contact face 45b formed inside the lock tube 45.

Further, a tapered face 45a whose diameter increases in the proximal end direction is formed on a proximal end inner circumferential face of the lock tube 45. The tapered face 45a of the lock tube 45 is in surface-contact with the tapered face 43 a of the adapter seizing portion 43.

In the state that the lock tube 45 is urged in the proximal end direction by the coil spring 46, the adapter seizing portion 43 is pressed by contact between the tapered face 43a formed on the outer circumferential face and the tapered face 45a of the lock tube 45 and elastically deforms in an inner diameter direction of the small diameter portion 42 of the assisting tool body 31.

Further, when the user slides the lock tube 45 to a distal end side against the urging force of the coil spring 46, the force of being pressed by contact between the tapered face 43a formed on the outer circumferential face and the tapered face 45a of the lock tube 45 decreases, and the adapter seizing portion 43 is restored in an outer diameter direction of the small diameter portion 42 of the assisting tool body 31 from the state of being elastically deformed and pressed in the inner diameter direction by the lock tube 45.

Since the lock tube 45 is continuously urged in the proximal end direction in the state that the optical adapter 20 is contained in the small diameter portion 42 of the assisting tool body 31, the assisting tool 30 configured in this way holds the optical adapter 20 by the holding face 43b of the adapter seizing portion 43 elastically deformed and pressed in the inner diameter direction of the small diameter portion 42 of the assisting tool body 31 by the lock tube 45 being in contact with the retaining ring 22 of the optical adapter 20, pressing the retaining ring 22.

The assisting tool 30 is configured to hold the optical adapter 20 in such a manner that the retaining ring 22 is prevented from running on idle against the rotation torque required at the time of screwing the retaining ring 22 of the optical adapter 20 to the distal end portion 11 of the endoscope 10, by the holding face 43b of the adapter seizing portion 43, on which the recesses and projections are formed, being engaged with the knurling 22a of the retaining ring 22 of the optical adapter 20. Note that, similarly to the second embodiment, the optical adapter 20 here also is in the configuration that recess portion 25 is not formed on the retaining ring 22.

Next, description will be made on a procedure for attaching and detaching the optical adapter 20 contained and held in the assisting tool 30 to and from the distal end portion 11 of the insertion portion 2 to change the optical characteristics of the endoscope 10, such as a view angle, such as close-up photography, wide-angle and enlargement (view from a distance), and an observation direction, by the assisting tool 30 of the present embodiment configured as described above at the time of using the endoscope 10.

First, in a state that the optical adapter 20 is contained and held in the assisting tool body 31 shown in Figs. 18 and 19, the assisting tool 30 is attached by the user so as to enclose the distal end portion 11 of the insertion portion 2 of the endoscope 10 from the opening 31a on the assisting tool body 31 side.

Then, the assisting tool 30 is rotated in the predetermined direction, and the optical adapter 20 is attached to the distal end portion 11 by the female screw portion 27 of the retaining ring 22 of the optical adapter 20 in the assisting tool body 31 and the male screw portion 14 of the distal end portion 11 being screwed to each other as shown in Fig. 20.

Note that, at the time of attaching the optical adapter 20 to the distal end portion 11, the holding face 43b of the adapter seizing portion 43 of the assisting tool 30 is in the state of being engaged with the knurling 22a of the retaining ring 22.

At this time, the adapter seizing portion 43 provided on the assisting tool body 31 of the assisting tool 30 is in the state of seizing the optical adapter 20, pressing the retaining ring 22 in the direction orthogonal to the direction of attaching and detaching the optical adapter 20 to and from the distal end portion 11. That is, the adapter seizing portion 43 presses and seizes the retaining ring 22 in the direction orthogonal to the direction of attaching and detaching the optical adapter 20 to and from the distal end portion 11.

Therefore, the optical adapter 20 is contained in the assisting tool body 31 of the assisting tool 30 with sufficient holding force without the retaining ring 22 engaged with the holding face 43b running on idle, against rotation torque at the time of screwing the retaining ring 22 of the optical adapter 20 and the distal end portion 11 of the endoscope 10 to each other.

Thereby, the user can certainly fasten the retaining ring 22 of the optical adapter 20 to the distal end portion 11 by rotating the assisting tool 30 in the predetermined direction.

In the assisting tool 30, when the lock tube 45 is slid in a distal end direction (a direction indicated by an arrow P in Fig. 20) against the urging force of the coil spring 46 in the above state, the adapter seizing portion 43 is restored in the outer circumferential direction of the small diameter portion 42 of the assisting tool body 31 and leaves the retaining ring 22 of the optical adapter 20.

Then, by the assisting tool 30 being pulled in the direction away from the distal end portion 11, the optical adapter 20 attached to the distal end portion 11 is caused to leave the assisting tool body 31 as shown in Fig. 21. Thus, the optical adapter 20 can be easily attached to the distal end portion 11 of the endoscope 10.

In this way, by the optical adapter 20 being attached to the distal end portion 11 of the insertion portion 2, the optical characteristics of the endoscope 10, such as a view angle, such as close-up photography, wide-angle and enlargement (view from a distance), and an observation direction are changed according to the kind of the attached optical adapter 20.

Note that, at the time of removing the optical adapter 20 from the distal end portion 11 of the insertion portion 2 of the endoscope 10, the optical adapter 20 attached to the distal end portion 11 is removed with use of the assisting tool 30 in a procedure opposite to the procedure at the time of attaching the optical adapter 20, and the optical adapter 20 is contained and held in the assisting tool body 31 of the assisting tool 30.

The assisting tool 30 of the present embodiment configured as described above has effects similar to those of the first and second embodiments and has an advantage that it is possible to prevent loss of the optical adapter 20 because the assisting tool 30 contains and holds the optical adapter 20 when the optical adapter 20 is not used without being attached to the distal end portion 11 of the endoscope 10.

Further, since the assisting tool 30 is configured so that the adapter seizing portion 43 holds the internal optical adapter 20, pressing the optical adapter 20, by the lock tube 45 receiving the urging force of the coil spring 46 and being urged in the proximal end direction, the coil spring 46, the lock tube 45 and the adapter seizing portion 43 do not easily wear out, deteriorate or the like even if the optical adapter 20 is repeatedly attached to and detached from the distal end portion 11 of the endoscope 10. Therefore, the assisting tool 30 is configured so that decrease in the force of holding the optical adapter 20 is prevented.

Therefore, the assisting tool 30 is configured such that the state of the adapter seizing portion 43 being engaged with the knurling 22a of the retaining ring 22, pressing the knurling 22a is kept, and the optical adapter 20 does not easily fall off from the assisting tool body 31. Thereby, it is possible to prevent loss of the optical adapter 20.

Furthermore, here also, by the force of holding the optical adapter 20 being prevented from decreasing, the amount of fastening or loosening force at the time of attaching or detaching the optical adapter 20 to or from the distal end portion 11 of the endoscope 10 becomes constant, and, therefore, the assisting tool 30 can stably attach or detach the optical adapter 20 to or from the distal end portion 11. Thereby, it does not happen that the retaining ring 22 of the optical adapter 20 loosens during an inspection, and the optical adapter 20 is prevented from falling off from the distal end portion 11 and dropping into an object.

From the above, similar to the first and second embodiments, the assisting tool 30 of the present embodiment is also configured to be capable of easily attaching the optical adapter 20 to the distal end portion 11 of the insertion portion 2 of the endoscope 10, and capable of attaching the optical adapter 20 to the distal end portion 11 with a stable amount of force, preventing the force of internally holding the optical adapter 20 from decreasing even if attachment and detachment of the optical adapter 20 is repeatedly performed.

### (Reference Examples)

Note that the assisting tool 30 may have a structure in which the optical adapter 20 is held and fixed in the assisting tool body 31, as described in reference examples below.

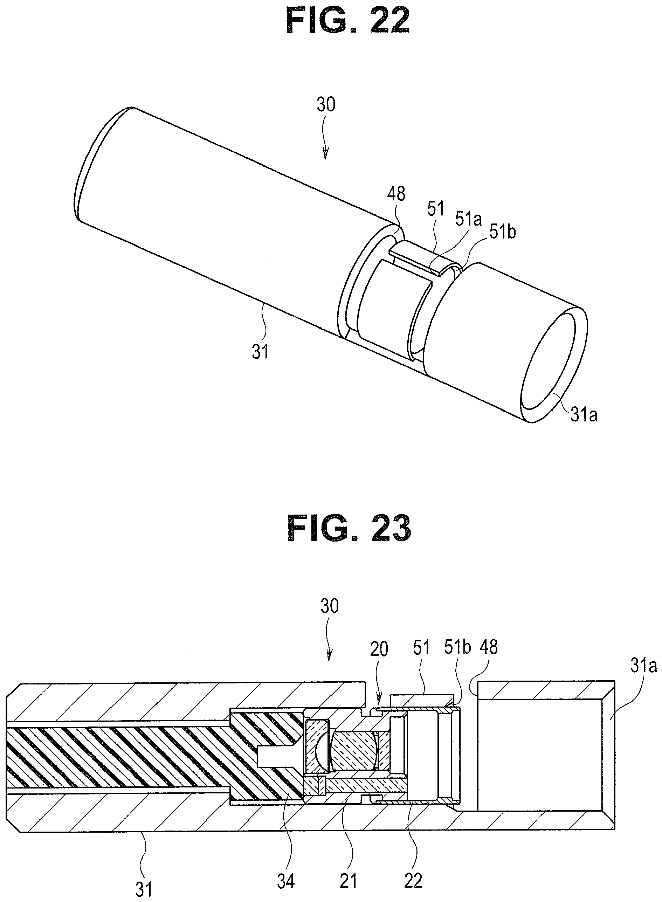

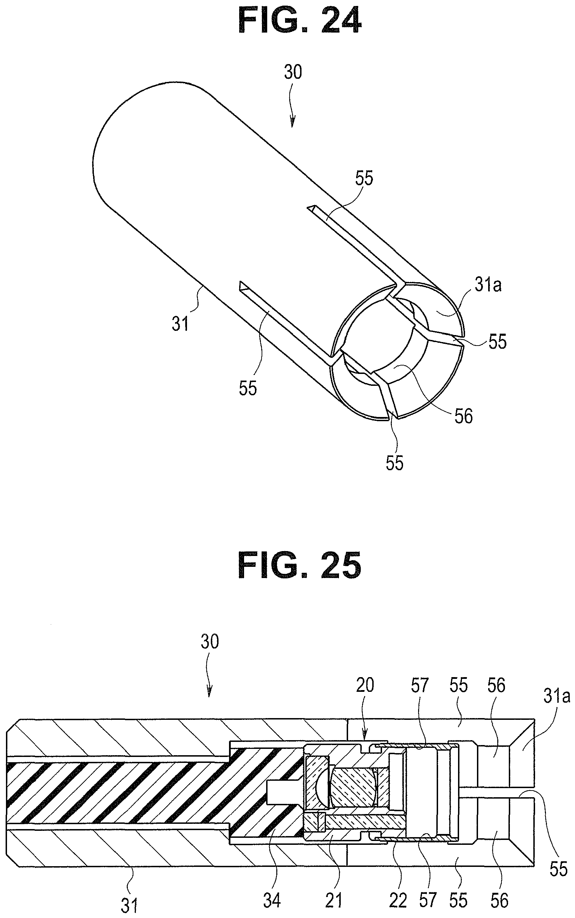

Fig. 22 is a perspective view showing a configuration of an endoscope optical adapter attachment/detachment assisting tool of a first reference example; Fig. 23 is a cross-sectional view showing the configuration of the endoscope optical adapter attachment/detachment assisting tool of the first reference example; Fig. 24 is a perspective view showing a configuration of an endoscope optical adapter attachment/detachment assisting tool of a second reference example; and Fig. 23 is a cross-sectional view showing the configuration of the endoscope optical adapter attachment/detachment assisting tool of the second reference example.

### (First Reference Example)

As shown in Figs. 22 and 23, in the assisting tool 30 of the present reference example, a notch portion 48 is formed on a midway portion of the assisting tool body 31, and an adapter seizing portion 51 in a form of a so-called C-shaped ring, which is a ring provided with a slit 51 a, is provided in the notch portion 48.

The adapter seizing portion 51 is formed integrally with the assisting tool body 31 with metal or rigid resin. Further, a tapered face 51b is formed on a proximal end side of the adapter seizing portion 51 where the optical adapter 20 is inserted.

In the assisting tool 30 configured as described above, by the optical adapter 20 being inserted from the opening 31a of the assisting tool body 31 and the optical adapter 20 coming into contact with the tapered face 51b of the adapter seizing portion 51, a diameter of the adapter seizing portion 51 increases.

Then, in the state that the optical adapter 20 is inserted until it comes into contact with the axial direction position restricting member 34, the adapter seizing portion 51 seizes the retaining ring 22 of the optical adapter 20, pressing the retaining ring 22 in the inner diameter direction orthogonal to the longitudinal direction. By such a configuration of the assisting tool 30 as described above, effects similar to those of each embodiment described above can be also obtained.

### (Second Reference Example)

As shown in Figs. 24 and 25, the assisting tool 30 of the present modification is provided with a plurality of slits 55, four slits 55 here, which are parallel to the longitudinal direction and formed at almost equal intervals around a circumference, from the proximal end to a midway portion of the assisting tool body 31.

In the assisting tool 30, an inward flange shaped retaining portion 56 for preventing the optical adapter 20 contained inside the assisting tool body 31 from falling off is projectingly formed in an inner diameter direction of a proximal end inner circumference portion of the assisting tool body 31. Further, in the assisting tool 30, a projection portion 57 as an inward flange shaped adapter seizing portion configured to hold the retaining ring 22 of the optical adapter 20, being in contact with the retaining ring 22, when the optical adapter 20 is contained inside the assisting tool 30 is projectingly formed on a midway inner circumferential portion in the inner diameter direction.

The assisting tool 30 configured as described above can cause the optical adapter 20 to be contained inside the assisting tool 30 or to leave from the inside by the diameter of the assisting tool body 31 being caused to increase by the four slits 55 of the assisting tool body 31.

Then, by the projection portion 57 formed on the assisting tool body 31 pressing the retaining ring 22 of the optical adapter 20 in the inner diameter direction orthogonal to the longitudinal direction, in the state that the optical adapter 20 is contained inside the assisting tool 30, the assisting tool 30 seizes the optical adapter 20 in the assisting tool body 31. By such a configuration of the assisting tool 30 as described above, effects similar to those of each embodiment described above can be also obtained.

Note that the assisting tool 30 described in the first to third embodiments described above may be in a configuration that the plurality of slits 55 parallel to the longitudinal direction are formed at almost equal intervals around the circumference of the assisting tool body 31 described in the present reference example, and the retaining portion 56 for preventing the optical adapter 20 contained inside the assisting tool body 31 from coming off is provided.

The inventions described in the respective embodiments above are not limited to the embodiments and the modifications. Additionally, it is possible to make various variations within a range not departing from the spirit of the inventions at implementation phase. Furthermore, each embodiment described above includes inventions at various stages, and various inventions can be extracted by appropriately combining a plurality of disclosed constituent features.

For example, even if some constituent features are deleted from all constituent features shown in each embodiment, a configuration obtained after deleting the constituent features can be extracted as an invention if the stated problems can be solved, and the stated advantageous effects can be obtained.

The present application is filed claiming priority based on Japanese Patent Application No. 2014-094882 filed in Japan on May 1, 2014, the disclosed content of which is incorporated in the specification, claims and drawings of the present application by reference.

## Claims

1. An endoscope optical adapter attachment/detachment assisting tool comprising:
a substantially cylindrical assisting tool body configured to contain and hold an optical adapter attachable to and detachable from a distal end portion of an insertion portion of an endoscope;
an adapter seizing portion configured to come into contact with the optical adapter contained in the assisting tool body and press the optical adapter in a direction orthogonal to a direction of attaching and detaching the optical adapter to and from the distal end portion to seize the optical adapter; and
an urging member configured to urge the adapter holding portion.

2. The endoscope optical adapter attachment/detachment assisting tool according to claim 1, wherein the adapter seizing portion has a projection portion to be engaged with a recess portion formed on the optical adapter.

3. The endoscope optical adapter attachment/detachment assisting tool according to claim 2, wherein, after the optical adapter is attached to the distal end portion, the adapter seizing portion comes off from the recess portion against urging force of the urging member, along a tapered face formed on a wall portion forming the recess portion of the optical adapter.

4. The endoscope optical adapter attachment/detachment assisting tool according to any one of claims 1 to 3, wherein the assisting tool body has a plurality of slits formed along a longitudinal direction and comprises a retaining portion projecting in an inner diameter direction near an opening through which the optical adapter is inserted.

## Amended claims

### Amended claims under Art. 19.1 PCT

**1.** (Amended) An endoscope optical adapter attachment/detachment assisting tool comprising:
a substantially cylindrical assisting tool body configured to contain and hold an optical adapter attachable to and detachable from a distal end portion of an insertion portion of an endoscope;
an adapter seizing portion configured to press and seize the optical adapter contained in the assisting tool body; and
an urging member configured to urge the adapter seizing portion in a direction orthogonal to a direction of attaching and detaching the optical adapter to and from the distal end portion.

**2.** The endoscope optical adapter attachment/detachment assisting tool according to claim 1, wherein the adapter seizing portion has a projection portion to be engaged with a recess portion formed on the optical adapter.

**3.** The endoscope optical adapter attachment/detachment assisting tool according to claim 2, wherein, after the optical adapter is attached to the distal end portion, the adapter seizing portion comes off from the recess portion against urging force of the urging member, along a tapered face formed on a wall portion forming the recess portion of the optical adapter.

**4.** (Amended) The endoscope optical adapter attachment/detachment assisting tool according to claim 1, wherein the assisting tool body has a plurality of slits formed along a longitudinal direction and comprises a retaining portion projecting in an inner diameter direction near an opening through which the optical adapter is inserted.

**5.** New) The endoscope optical adapter attachment/detachment assisting tool according to claim 1, wherein the assisting tool body comprises an axial direction position restricting member configured to determine an axial direction position of the optical adapter.

**6.** New) The endoscope optical adapter attachment/detachment assisting tool according to claim 1, wherein the urging member urges the adapter seizing portion so that the adapter seizing portion projects from an inner circumferential face of the assisting tool body by a predetermined length.

**7.** New) The endoscope optical adapter attachment/detachment assisting tool according to claim 1, wherein the urging member urges the adapter seizing member so that holding force larger than a rotation torque required at time of screwing the optical adapter and the distal end portion to each other is generated.

**8.** New) The endoscope optical adapter attachment/detachment assisting tool according to claim 1, wherein the urging member is provided in a hole portion formed from an outer circumferential portion of the assisting tool body in an inner diameter center direction.

**9.** New) The endoscope optical adapter attachment/detachment assisting tool according to claim 1, wherein the urging member comprises an operation portion provided in a state of being rotatably held along a longitudinal direction of the assisting tool body and configured to operate the adapter seizing member.
